(19) Europäisches Patentamt
European Patent Office
Office européen des brevets

(11) EP 0 705 598 B1

# (12) FASCICULE DE BREVET EUROPEEN

(45) Date de publication et mention de la délivrance du brevet:
**17.11.1999 Bulletin 1999/46**

(51) Int. Cl.$^6$: **A61K 7/13**

(21) Numéro de dépôt: **95402040.0**

(22) Date de dépôt: **08.09.1995**

(54) **Composition de teinture d'oxydation des fibres kératiniques comprenant une association d'au moins deux dérivés particuliers de paraphénylènediamine, et utilisation**

Oxydationsfärbemittel für keratinische Fasern mit einer Zusammensetzung mindestens zwei Paraphenylendiamin Derivate und deren Verwendung

Oxidation dye composition for keratinic fibers comprising an association of at least two paraphenylenediamine derivatives, and their use

(84) Etats contractants désignés:
**AT BE CH DE ES FR GB IT LI NL SE**

(30) Priorité: **07.10.1994 FR 9412003**

(43) Date de publication de la demande:
**10.04.1996 Bulletin 1996/15**

(73) Titulaire: **L'OREAL**
**75008 Paris (FR)**

(72) Inventeurs:
- **Cotteret, Jean**
  **F-78480 Verneuil sur Seine (FR)**
- **Audousset, Marie-Pascale**
  **F-92600 Asnieres (FR)**

(74) Mandataire: **Goulard, Sophie et al**
**L'OREAL**
**Département Propriété Industrielle**
**6, rue Bertrand Sincholle**
**F-92585 Clichy Cedex (FR)**

(56) Documents cités:
**EP-A- 0 459 901**
**FR-A- 2 156 527**
**FR-A- 2 364 888**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 705 598 B1

Printed by Xerox (UK) Business Services
2.16.7/3.6

## Description

La présente invention concerne une composition de teinture d'oxydation des fibres kératiniques et en particulier des fibres kératiniques humaines, comprenant au moins une association d'au moins deux dérivés particuliers de la paraphénylènediamine et, le cas échéant, un ou plusieurs coupleurs.

Elle concerne également l'utilisation d'une telle composition dans l'application cosmétique susmentionnée.

Il est connu de teindre les fibres kératiniques et en particulier les cheveux humains, avec des compositions de teinture contenant des précurseurs de colorants d'oxydation, en particulier des ortho- ou para-phénylénediamines, des ortho- ou para-aminophénols, généralement appelés "bases d'oxydation", et des coupleurs encore appelés modificateurs de coloration, plus particulièrement des métaphénylènediamines, des méta-aminophénols et des métadiphénols, qui permettent de modifier et d'enrichir de reflets les colorations "de fond" obtenues par les produits de condensation des bases d'oxydation.

On recherche, dans le domaine de la teinture capillaire d'oxydation, des précurseurs de colorants d'oxydation et des coupleurs capables d'engendrer, lorsqu'ils sont associés, des nuances ayant une résistance satisfaisante à la lumière, aux lavages, aux intempéries, à la transpiration et aux différents traitements capillaires que peuvent subir les cheveux.

Jusqu'ici ces nuances ont été obtenues avec des teintures à base de paraphénylènediamine. Mais actuellement on cherche à remplacer la paraphénylènediamine. Dans cette optique, on a déjà proposé, notamment dans les demandes de brevet EP 0 459 901, FR-A-2 156 527 et FR-A-2 364 888, d'utiliser des paraphénylènediamines substituées sur le noyau benzénique ou sur une fonction amine.

Cependant, il est apparu à la demanderesse que, pour être encore plus satisfaisantes, les teintures ci-dessus devaient être moins sélectives, c'est à dire qu'elles devaient être moins sensibles, sur le plan tinctorial, aux divers degrés de sensibilisation des cheveux à teindre, afin que l'écart de couleur observé sur ces cheveux plus ou moins sensibilisés, soit le plus faible possible, et qu'ainsi la chevelure soit teinte uniformément.

Or, après d'importantes recherches menées sur la question, la demanderesse vient maintenant de découvrir qu'il est possible d'obtenir des teintures d'oxydation à base de dérivés de la paraphénylènediamine qui présentent une sélectivité nettement améliorée par rapport à celles jusqu'ici connues, lorsqu'on utilise une des associations suivantes :

- la 2-(β-hydroxyéthyl) paraphénylènediamine et la 2,6-dimethylparaphénylène-diamine ;
- la 2-(β-hydroxyéthyl) paraphénylènediamine et la N,N-di-(β-hydroxyéthyl) paraphénylène-diamine ;
- la 2-(β-hydroxyéthyl) paraphénylènediamine et la N-(β-méthoxyéthyl) paraphénylène-diamine ;
- la 2,6-diméthylparaphénylènediamine et la N-(β-méthoxyéthyl) paraphénylènediamine ;
- la 2,3-diméthylparaphénylènediamine et la N,N-di-(β-hydroxyéthyl) paraphénylène-diamine ;
- la 2,3-diméthylparaphénylènediamine, la N,N-di-(β-hydroxyéthyl) paraphénylènediamine et la 2,6-diméthylparaphénylènediamine ;
- la 2-(β-hydroxyéthyl) paraphénylènediamine, la 2,6-diméthylparaphénylènediamine et la 2,3-diméthylparaphénylènediamine ;
- la 2-(β-hydroxyéthyl) paraphénylènediamine, la 2,6-diméthylparaphénylènediamine et la N,N-di-(β-hydroxyéthyl) paraphénylènediamine ;
- la 2,6-diéthylparaphénylènediamine et la N,N-di-(β-hydroxyéthyl) paraphénylènediamine ;
  et leurs sels d'addition avec un acide.

Cette découverte est à la base de la présente invention.

La présente invention a ainsi pour objet une composition de teinture d'oxydation pour fibres kératiniques, en particulier pour fibres kératiniques humaines, telles que les cheveux, du type comprenant, dans un milieu approprié pour la teinture, au moins un précurseur de colorant d'oxydation (base d'oxydation) et, le cas échéant, un ou plusieurs coupleurs, qui est caractérisée par le fait qu'elle contient, à titre de précurseur de colorant d'oxydation, au moins une des associations suivantes :

- la 2-(β-hydroxyéthyl) paraphénylènediamine et la 2,6-diméthylparaphénylènediamine ;
- la 2-(β-hydroxyéthyl) paraphénylènediamine et la N,N-di-(β-hydroxyéthyl) paraphénylène-diamine ;
- la 2-(β-hydroxyéthyl) paraphénylènediamine et la N-(β-méthoxyéthyl) paraphénylène-diamine ;
- la 2,6-diméthylparaphénylènediamine et la N-(β-méthoxyéthyl) paraphénylènediamine ;
- la 2,3-diméthylparaphénylènediamine et la N,N-di-(β-hydroxyéthyl) paraphénylène-diamine ;
- la 2,3-diméthylparaphénylènediamine, la N,N-di-(β-hydroxyéthyl) paraphénylènediamine et la 2,6-diméthylparaphénylènediamine ;
- la 2-(β-hydroxyéthyl) paraphénylènediamine, la 2,6-diméthylparaphénylènediamine et la 2,3-diméthylparaphénylènediamine ;

- la 2-(β-hydroxyéthyl) paraphénylènediamine, la 2,6-diméthylparaphénylènediamine et la N,N-di-(β-hydroxyéthyl) paraphénylènediamine ;
- la 2,6-diéthylparaphénylènediamine et la N,N-di-(β-hydroxyéthyl) paraphénylènediamine ;
  et leurs sels d'addition avec un acide.

Les nouvelles teintures obtenues dans le cadre de la présente invention permettent d'aboutir à des colorations moins sélectives et donc plus uniformes de la racine à la pointe des cheveux.

Elles présentent en outre une résistance satisfaisante aux agressions extérieures (lumière et pluie), à la transpiration, et aux différents traitements cosmétiques capillaires (permanente et shampooings).

Un autre objet de la présente invention porte sur une composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, au moins une association de précurseurs de colorant d'oxydation telle qu'elle a été définie ci-avant et, le cas échéant, un ou plusieurs coupleurs, la couleur étant révélée à pH alcalin, neutre ou acide à l'aide d'un agent oxydant qui est mélangé juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier compartiment contient au moins une association d'au moins deux dérivés de la paraphénylènediamine listés ci-avant à titre de précurseur de colorant d'oxydation et, le cas échéant un ou plusieurs coupleurs, et le deuxième compartiment un agent oxydant.

Mais d'autres caractéristiques, aspects, objets et avantages de l'invention apparaîtront encore plus clairement à la lecture de la description et des exemples qui suivent.

Les sels d'acide des dérivés de la paraphénylènediamine qui peuvent être utilisés selon l'invention sont choisis de préférence parmi les chlorhydrates, les sulfates, les bromhydrates et les tartrates.

La concentration en chacun des dérivés de la paraphénylènediamine de l'association ou leurs sels peut varier entre 0,01 et 10 % en poids environ par rapport au poids total de la composition de teinture et de préférence entre 0,05 et 8 % environ de ce poids.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11 environ, qui peut être ajusté à la valeur choisie au moyen soit d'agents alcalinisants habituellement utilisés en teinture des fibres kératiniques, tels que l'ammoniaque, les carbonates alcalins, les alcanolamines, par exemple les mono-, di- et triéthanolamine ainsi que leurs dérivés, les hydroxydes de sodium ou de potassium et les composés de formule :

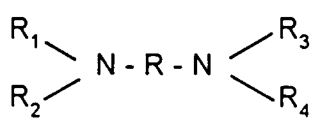

dans laquelle R est un reste propylène éventuellement substitué par un groupement hydroxyle ou un radical alkyle en $C_1$-$C_4$ ; $R_1$ $R_2$, $R_3$ et $R_4$, simultanément ou indépendamment l'un de l'autre, représentent un atome d'hydrogène, un radical alkyle en $C_1$-$C_4$ ou hydroxyalkyle en $C_1$-$C_4$, soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus est tel qu'après un mélange avec la composition (A), le pH de la composition appliquée sur les fibres kératiniques humaines varie de préférence entre 3 et 11 environ. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 2 à 40 minutes, de préférence 5 à 30 minutes, après quoi on rince, on lave au shampooing, on rince à

nouveau et on sèche.

Comme indiqué précédemment, les compositions de teinture préférées selon l'invention peuvent contenir en outre, à côté des précurseurs de colorants d'oxydation définis ci-dessus, un ou plusieurs coupleurs.

Parmi ces coupleurs, on peut citer plus particulièrement : le 5-amino 2-méthyl phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyl)amino 1-méthoxy benzène, le sésamol, le 6-hydroxy indole, le 4-hydroxybenzimidazole, la 6-hydroxy 1,4-benzoxazine, le 4-amino 1,2-méthylènedioxy benzène, et le 1-(β-hydroxyéthyl)amino 3,4-méthylènedioxy benzène. On peut mettre en oeuvre d'autres coupleurs usuels.

Pour modifier les nuances ou les enrichir en reflets, les compositions de teinture peuvent encore contenir, en plus des précurseurs de colorants d'oxydation définis ci-dessus et, le cas échéant, des coupleurs associés, des précurseurs de colorants d'oxydation complémentaires choisis parmi le 2-aminophénol, le 4-aminophénol, le 4-amino 3-méthyl phénol, le 4-amino 3-hydroxyméthyl phénol et le 4-amino 3-fluoro phénol, ainsi que des colorants directs choisis parmi les colorants nitrés, azoïques ou anthraquinoniques.

Leur concentration pondérale peut varier entre 0,0005 et 10 % environ, et de préférence entre 0,001 et 5 % environ, par rapport au poids total de la composition de teinture appliquée sur les cheveux.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tert.butyl hydroquinone et l'acide homogentisique, et ils sont alors généralement présents dans des proportions comprises entre environ 0,05 et 1,5 % en poids par rapport au poids total de la composition.

Les compositions de teinture selon l'invention contiennent également, dans leur forme de réalisation préférée, des agents tensioactifs bien connus de la technique, dans des proportions comprises entre environ 0,5 et 55 % en poids, et de préférence entre 2 et 50 % en poids par rapport au poids total de la composition, des solvants organiques, dans des proportions comprises entre environ 1 et 40 % en poids, et en particulier entre environ 5 et 30 % en poids par rapport au poids total de la composition, ou tout autre adjuvant cosmétiquement acceptable et connu de la technique antérieure en teinture d'oxydation capillaire.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés.

Dans ces exemples, le critère de sélectivité de la teinture est évalué au moyen de l'indice de variation de couleur **I** calculé selon l'équation de NICKERSON suivante (voir à cet égard "Journal of the Optical Society of America ", 1944, Sept., Vol 34, n° 9, pp 550-570) :

$$I = 0,4\, C_0\, \Delta H + 6 \Delta V + 3 \Delta C$$

dans laquelle les paramètres H, V, et C représentent les paramètres de la notation MUNSELL (Norme ASTM D 1535-68) qui définit la couleur, H désignant la nuance ou HUE, V désignant l'intensité ou VALUE, et C la pureté ou CHROMATICITE ; $C_0$ désigne la pureté de la mèche par rapport à laquelle on désire évaluer la différence de couleur Les mesures sont effectuées sur un colorimètre MINOLTA CM 2002.

**EXEMPLE**

On a préparé la composition de teinture, conforme à l'invention, suivante :

| | |
|---|---|
| - 2-(β-hydroxyéthyl) paraphénylènediamine, dichlorhydrate | 0,5625 g. |
| - 2,6-diméthylparaphénylènediamine, dichlorhydrate | 0,5225 g |
| - Résorcine | 0,55 g |
| - Alcool oléique polyglycérolé à 4 moles de glycérol à 78 % de de matière active (M.A.) | 5,7 g M.A. |

(suite)

| | |
|---|---|
| - Alcool oléique polyglycérolé à 2 moles de glycérol | 4,0 g |
| - Acide oléique | 3,0 g |
| - Amine oléique à 2 moles d'oxyde d'éthylène, vendue sous la dénomination Ethomeen 012 par la Société AKZO | 7,0 g |
| - Laurylamino succinamate de diéthylaminopropyle, sel de sodium à 55% de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de M.A. | 0,46 g M.A. |
| - Acétate d'ammonium | 0,8 g |
| - Antioxydant, séquestrant, | qs |
| - Parfum, conservateur, | qs |
| - Solution aqueuse d'ammoniaque à 20 % de $NH_3$ | 2,0 g M.A. |
| - Eau déminéralisée q.s.p. | 100,0 g |

On a réalisé, parallèlement, deux compositions comparatives (A) et (B) ne faisant pas partie de l'invention qui, en remplacement de l'association des deux dérivés de la paraphénylènediamine : 2-(β-hydroxyéthyl) paraphénylènediamine et 2,6-diméthyl para-phénylènediamine, contenaient :

<u>*Composition comparative (A)*</u> :

1,125 g de 2-(β-hydroxyéthyl) paraphénylènediamine, dichlorhydrate

<u>*Composition comparative (B)*</u> :

1,045 g de 2,6-diméthylparaphénylènediamine, dichlorhydrate

Dans ces deux compositions comparatives, la concentration en dérivé de paraphénylènediamine est égale à l'équivalent molaire de la concentration totale des deux dérivés de paraphénylènediamine de l'association conforme à l'invention. Les autres constituants des compositions comparatives (A) et (B) étaient identiques, en nature et en concentration à ceux de la composition conforme à l'invention.

Au moment de l'emploi, on a mélangé chacune des trois compositions, celle de l'invention, et les compositions comparatives (A) et (B) ne faisant pas partie de l'invention, poids pour poids avec de l'eau oxygénée titrant 20 volumes (6% en poids), de pH 3.

On a obtenu trois mélanges de pH 9,8.

On a appliqué, pendant 30 minutes, chacun de ces mélanges sur des mèches de cheveux gris à 90 % de blancs **non permanentés** d'une part, ainsi que sur des mèches de cheveux gris à 90 % de blancs **permanentés** d'autre part. Les cheveux ont ensuite été rincés, lavés au shampooing, rincés et séchés.

On a ensuite apprécié, et comparé, les critères de sélectivité de teinture attachés aux compositions obtenues.

Avant teinture, les cheveux gris à 90 % de blancs **non permanentés** présentaient une nuance MUNSELL de : 4,2 Y 5,5 / 1,7.

Avant teinture, les cheveux gris à 90 % de blancs **permanentés** présentaient une nuance MUNSELL de : 4,0 Y 5,5 / 1,7.

Les nuances obtenues après teinture avec chacune des compositions figurent dans le tableau ci-après :

| Composition | Couleur sur cheveux non permanentés | Couleur sur cheveux permanentés |
|---|---|---|
| | | |
| Invention | 1,2 Y 4,6/2,2 | 1,5 Y 4,3/2,3 |
| A | 1,7 Y 4,4/2,2 | 1,7 Y 4,1/2,5 |
| B | 1,1 Y 4,4/2,3 | 1,1 Y 3,7/2,3 |

Les différences de montée de la coloration, exprimées par l'équation de NICKERSON, **entre les cheveux permanentés et les cheveux non permanentés** (cette montée étant elle-même exprimée, dans chacun des cas, par l'écart de coloration par rapport à une mèche non teinte), étaient les suivantes :

- **Composition conforme à l'invention :1,76**
- **Composition comparative (A) : 2,56**
- **Composition comparative (B) : 4,06**

Ces résultats démontrent qu'une chevelure permanentée et comportant des racines non permanentées, présentera, après teinture avec la composition selon l'invention contenant l'association de deux dérivés de paraphénylènediamine, une coloration plus uniforme qu'avec les teintures réalisées avec les compositions comparatives (A) ou (B) ne faisant pas partie de l'invention car ne contenant chacune qu'un seul des deux dérivés de paraphénylènediamine utilisés dans la composition de l'invention.

**Revendications**

1. Composition de teinture d'oxydation pour fibres kératiniques, en particulier pour fibres kératiniques humaines telles que les cheveux, du type comprenant dans un milieu approprié pour la teinture, au moins un précurseur de colorant d'oxydation et, le cas échéant, un ou plusieurs coupleurs, caractérisée par le fait qu'elle contient, à titre de précurseur de colorant d'oxydation, au moins une des associations suivantes :

   - la 2-(β-hydroxyéthyl) paraphénylènediamine et la 2,6-diméthylparaphénylènediamine ;
   - la 2-(β-hydroxyéthyl) paraphénylènediamine et la N,N-di-(β-hydroxyéthyl) paraphénylènediamine ;
   - la 2-(β-hydroxyéthyl) paraphénylènediamine et la N-(β-méthoxyéthyl) paraphénylènediamine ;
   - la 2,6-diméthylparaphénylènediamine et la N-(β-méthoxyéthyl) paraphénylènediamine ;
   - la 2,3-diméthylparaphénylènediamine et la N,N-di-(β-hydroxyéthyl) paraphénylènediamine ;
   - la 2,3-diméthylparaphénylènediamine, la N,N-di-(β-hydroxyéthyl) paraphénylènediamine et la 2,6-diméthylparaphénylènediamine ;
   - la 2-(β-hydroxyéthyl) paraphénylènediamine, la 2,6-diméthylparaphénylènediamine et la 2,3-diméthylparaphénylènediamine ;
   - la 2-(β-hydroxyéthyl) paraphénylènediamine, la 2,6-diméthylparaphénylènediamine et la N,N-di-(β-hydroxyéthyl) paraphénylènediamine ;
   - la 2,6-diéthylparaphénylènediamine et la N,N-di-(β-hydroxyéthyl) paraphénylènediamine ;
   et leurs sels d'addition avec un acide.

2. Composition selon la revendication 1, caractérisée par le fait que les sels d'addition avec un acide des dérivés de la paraphénylènediamine sont choisis parmi les chlorhydrates, les sulfates, les bromhydrates et les tartrates.

3. Composition selon la revendication 1 ou 2, caractérisée par le fait que chacun des dérivés de la paraphénylènediamine de l'association ou ses sels, est présent dans une concentration comprise entre 0,01 et 10 % en poids par rapport au poids total de la composition, et de préférence entre 0,05 et 8 % en poids.

4. Composition selon l'une quelconque des revendications précédentes, caractérisée par le fait qu'elle contient en outre un ou plusieurs coupleurs.

5. Composition selon l'une quelconque des revendications précédentes, caractérisée par le fait qu'elle contient en outre des colorants directs choisis parmi les colorants nitrés, azoïques ou anthraquinoniques et/ou des précurseurs de colorants d'oxydation complémentaires choisis parmi le 2-aminophénol, le 4-aminophénol, le 4-amino 3-méthyl phénol, le 4-amino 3-hydroxyméthyl phénol et le 4-amino 3-fluoro phénol.

6. Composition selon l'une quelconque des revendications précédentes, prête à l'emploi, caractérisée par le fait qu'elle contient en outre un agent oxydant et qu'elle possède un pH compris entre 3 et 11.

7. Procédé de teinture des fibres kératiniques et en particulier des fibres kératiniques humaines telles que les cheveux, caractérisé par le fait qu'il consiste à appliquer sur les fibres une composition de teinture (A) telle que définie à l'une quelconque des revendications 1 à 5, et à révéler la couleur en milieu alcalin, neutre ou acide à l'aide d'un agent oxydant qui est ajouté juste au moment de l'emploi à cette composition (A) ou qui est présent dans une composition (B) appliquée simultanément ou séquentiellement de façon séparée.

8. Dispositif à plusieurs compartiments ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A) telle que définie à l'une quelconque des revendications 1 à 5, et un autre une composition (B) comprenant un agent oxydant dans un milieu approprié pour la teinture.

9. Utilisation d'une composition de teinture d'oxydation telle que définie à l'une quelconque des revendications 1 à 6 ou d'un dispositif de teinture ou "Kit" à plusieurs compartiments tel que défini à la revendication 8, pour la teinture des fibres kératiniques et en particulier des fibres kératiniques humaines telles que les cheveux.

## Claims

1. Oxidation dye composition for keratin fibres, in particular for human keratin fibres such as the hair, of the type comprising, in a medium which is suitable for dyeing, at least one oxidation dye precursor and, where appropriate, one or more couplers, characterized in that it contains, as oxidation dye precursor, at least one of the following combinations:

   - 2-(β-hydroxyethyl)-para-phenylenediamine and 2,6-dimethyl-para-phenylenediamine;
   - 2-(β-hydroxyethyl)-para-phenylenediamine and N,N-di(β-hydroxyethyl)-para-phenylenediamine;
   - 2-(β-hydroxyethyl)-para-phenylenediamine and N-(β-methoxyethyl)-para-phenylenediamine;
   - 2,6-dimethyl-para-phenylenediamine and N-(β-methoxyethyl)-para-phenylenediamine;
   - 2,3-dimethyl-para-phenylenediamine and N,N-di(β-hydroxyethyl)-para-phenylenediamine;
   - 2,3-dimethyl-para-phenylenediamine, N,N-di(β-hydroxyethyl)-para-phenylenediamine and 2,6-dimethyl-para-phenylenediamine;
   - 2-(β-hydroxyethyl)-para-phenylenediamine, 2,6-dimethyl-para-phenylenediamine and 2,3-dimethylpara-phenylenediamine;
   - 2-(β-hydroxyethyl)-para-phenylenediamine, 2,6-dimethyl-para-phenylenediamine and N,N-di(β-hydroxyethyl)-para-phenylenediamine;
   - 2,6-diethyl-para-phenylenediamine and N,N-di(β-hydroxyethyl)-para-phenylenediamine;

   and the addition salts thereof with an acid.

2. Composition according to Claim 1, characterized in that the addition salts with an acid of the paraphenylenediamine derivatives are chosen from the hydrochlorides, the sulphates, the hydrobromides and the tartrates.

3. Composition according to Claim 1 or 2, characterized in that each of the para-phenylenediamine derivatives or salts thereof in the combination is present in a concentration between 0.01 and 10% by weight relative to the total weight of the composition, and preferably between 0.05 and 8% by weight.

4. Composition according to any one of the preceding claims, characterized in that it also contains one or more couplers.

5. Composition according to any one of the preceding claims, characterized in that it also contains direct dyes chosen from nitro dyes, azo dyes or anthraquinone dyes and/or additional oxidation dye precursors chosen from 2-aminophenol, 4-aminophenol, 4-amino-3-methylphenol, 4-amino-3-hydroxymethylphenol and 4-amino-3-fluorophenol.

6. Composition according to any one of the preceding claims, which is a ready-to-use composition, characterized in that it also contains an oxidizing agent and in that it has a pH between 3 and 11.

7. Process for dyeing keratin fibres and in particular human keratin fibres such as the hair, characterized in that it consists in applying to the fibres a dye composition (A) as defined in any one of Claims 1 to 5, and in developing the colour in alkaline, neutral or acidic medium with the aid of an oxidizing agent which is added to this composition (A) at the time of use or which is present in a composition (B) applied simultaneously or sequentially in a separate manner.

8. Multi-compartment device or "kit" for dyeing keratin fibres and in particular human keratin fibres such as the hair, characterized in that it contains at least two compartments, one of which includes a composition (A) as defined in any one of Claims 1 to 5, and the other of which includes another composition (B) comprising an oxidizing agent in a medium which is suitable for dyeing.

9. Use of an oxidation dye composition as defined in any one of Claims 1 to 6 or of a multi-compartment dyeing device or "kit" as defined in Claim 8, for dyeing keratin fibres and in particular human keratin fibres such as the hair.

**Patentansprüche**

1. Zusammensetzung zum oxidativen Färben von Keratinfasern und insbesondere menschlichen Keratinfasern, wie der Haare, die in einem zum Färben geeigneten Medium mindestens ein Farbstoffvorprodrnkt eines Oxidationsfarbstoffes und gegebenenfalls einen oder mehrere Kuppler enthält, dadurch gekennzeichnet, daß sie als Farbstoffvorprodukt eines Oxidationsfarbstoffes mindestes eine der folgenden Kombinationen enthält:

   - 2-(β-Hydroxyethyl)-p-phenylendiamin und 2,6-Dimethyl-p-phenylendiamin;
   - 2-(β-Hydroxyethyl)-p-phenylendiamin und N,N-di-(β-Hydroxyethyl)-p-phenylendiamin;
   - 2-(β-Hydroxyethyl)-p-phenylendiamin und N-(β-Methoxyethyl)-p-phenylendiamin;
   - 2,6-Dimethyl-p-phenylendiamin und N-(β-Methoxyethyl)-p-phenylendiamin;
   - 2,3-Dimethyl-p-phenylendiamin und N,N-di-(β-Hydroxyethyl)-p-phenylendiamin;
   - 2,3-Dimethyl-p-phenylendiamin, N,N-di-(β-Hydroxyethyl)-p-phenylendiamin und 2,6-Dimethyl-p-phenylendiamin;
   - 2-(β-Hydroxyethyl)-p-phenylendiamin, 2,6-Dimethyl-p-phenylendiamin und 2,3-Dimethyl-p-phenylendiamin;
   - 2-(β-Hydroxyethyl)-p-phenylendiamin, 2,6-Dimethyl-p-phenylendiamin und N,N-di-(β-Hydroxyethyl)-p-phenylendiamin;
   - 2,6-Diethyl-p-phenylendiamin und N,N-di-(β-Hydroxyethyl)-p-phenylendiamin;

   sowie die Additionssalze dieser Verbindungen mit einer Säure.

2. Zusammensetzung nach Anspruch 1, dadurch gekennzeichnet, daß die Additionssalze der p-Phenylendiaminderivate mit einer Säure unter den Hydrochloriden, Sulfaten, Hydrobromiden und Tartraten ausgewählt sind.

3. Zusammensetzung nach Anspruch 1 oder 2, dadurch gekennzeichnet, daß jedes der p-Phenylendiaminderivate der Kombination oder ihre Salze in einer Konzentration im Bereich von 0,01 bis 10 Gew.-%, bezogen auf das Gesamtgewicht der Zusammensetzung, und vorzugsweise im Bereich von 0,05 bis 8 Gew.-% vorliegen.

4. Zusammensetzung nach einem der vorhergehenden Ansprüche, dadurch gekennzeichnet, daß sie ferner einen oder mehrere Kuppler enthält.

5. Zusammensetzung nach einem der vorhergehenden Ansprüche, dadurch gekennzeichnet, daß sie ferner einen oder mehrere Direktfarbstoffe, die unter den Nitrofarbstoffen, Azofarbstoffen und Anthrachinonen ausgewählt sind, und/oder zusätzliche Farbstoffvorprodukte von Oxidationsfarbstoffen, die unter 2-Aminophenol, 4-Aminophenol, 4-Amino-3-methylphenol, 4-Amino-3-hydroxymethylphenol und 4-Amino-3-fluorphenol ausgewählt sind, enthält.

6. Zusammensetzung nach einem der vorhergehenden Ansprüche, die gebrauchsfertig ist, dadurch gekennzeichnet, daß sie ferner ein Oxidationsmittel enthält und daß sie einen pH-Wert im Bereich von 3 bis 11 aufweist.

7. Verfahren zum Färben von Keratinfasern und insbesondere menschlichen Keratinfasern, wie dem Haar, dadurch gekennzeichnet, daß es darin besteht, auf die Fasern eine Färbemittelzusammensetzung (A) nach einem der Ansprüche 1 bis 5 aufzutragen und die Farbe in einem alkalischen, neutralen oder sauren Medium mit einem Oxidationsmittel zu entwickeln, das bei der Anwendung zu der Zusammensetzung (A) gegeben wird oder das in einer Zusammensetzung (B) vorliegt, die gleichzeitig oder getrennt davon anschließend aufgetragen wird.

**8.** Vorrichtung mit mehreren Abteilungen oder Kit zum Färben von Keratinfasern und insbesondere menschlichen Keratinfasern, wie dem Haar, dadurch gekennzeichnet, daß es mindestens zwei Abteilungen aufweist, wobei eine Abteilung die Zusammensetzung (A) nach einem der Ansprüche 1 bis 5 und eine zweite Abteilung die Zusammensetzung (B) enthält, die in einem zum Färben geeigneten Medium ein Oxidationsmittel enthält.

**9.** Verwendung einer Zusammensetzung zum oxidativen Färben nach einem der Ansprüche 1 bis 6 oder einer Vorrichtung oder eines Kits zum Färben mit mehreren Abteilungen nach Anspruch 8 zum Färben von Keratinfasern und insbesondere von menschlichen Keratinfasern, wie dem Haar.